# EUROPEAN PATENT SPECIFICATION

(11) **EP 2 641 543 B1**
(45) Date of publication and mention of the grant of the patent: **04.03.2015**
(21) Application number: 12854673.6
(22) Date of filing: 11.09.2012
(51) Int. Cl.: A61B 8/12, A61B 8/00, G01N 29/00

(54) **ULTRASOUND PROBE**
ULTRASCHALLSONDE
SONDE ULTRASONORE

(30) Priority: 08.12.2011 JP 2011269204
(43) Date of publication of application: 25.09.2013
(73) Proprietor: Olympus Medical Systems Corp., Tokyo 151-0072 (JP)
(72) Inventor: KURIHARA, Kozue, Tokyo 151-0072 (JP)
(74) Representative: Schicker, Silvia
(86) International application number: PCT/JP2012/073178
(87) International publication number: WO 2013/084555

(56) References cited:
- EP-A2- 0 659 387
- WO-A1-2008/115151
- WO-A1-2011/028181
- JP-A- 2003 070 791
- JP-A- 2003 325 524
- JP-A- 2007 135 949
- US-A- 4 224 929
- US-A- 6 142 945
- US-A1- 2003 004 434
- US-A1- 2005 154 308
- US-A1- 2007 049 796

## Description

### Technical Field

The present invention relates to a structure of a balloon fixing portion for mounting and fixing an ultrasound balloon which covers an ultrasound transmission/reception portion of an ultrasound probe.

### Background Art

Conventionally, an ultrasound endoscope system has been widely spread, which is configured to insert an ultrasound probe, which includes an insertion section that incorporates at a distal end thereof an ultrasound transmission/reception portion including an ultrasound transducer, into a body, and to generate an ultrasound image by transmitting ultrasound toward a living tissue from the ultrasound transmission/reception portion and receiving the ultrasound reflected from the living tissue.

When ultrasound diagnosis is performed by using such a kind of ultrasound endoscope system, a medium that effectively transmits ultrasound is allowed to intervene between the ultrasound transmission/reception portion of the ultrasound probe and a living tissue, in order to acquire an excellent ultrasound image. An ultrasound endoscope system has been commercialized, which is configured such that an ultrasound balloon made of an elastic material is mounted to a distal end section of an insertion section of an ultrasound probe and an ultrasound transmitting medium (liquid or the like) is filled in the balloon.

As disclosed in Japanese Patent Application Laid-Open Publication Nos. 2010-005098 and 2010-017485, for example, a conventional ultrasound probe generally includes a balloon formed by including opening portions made of thick ring-shape portions at both ends of a cylindrical elastic member, and a distal end section of an insertion section which corresponds to the balloon, that is, groove portions for fixing the balloon, which are respectively formed on a proximal end side and a distal end side across an ultrasound transmission/reception portion.

In order to mount the balloon to the distal end section of the insertion section, an operation for attaching the balloon is performed such that the ring-shape portions of the balloon are attached to balloon fixing grooves so as to fit the ring-shape portions therein by using a balloon mounting jig, for example.

After the balloon is thus mounted at a predetermined site of the distal end section of the ultrasound probe, an inspection is performed by filling an ultrasound transmitting medium in the balloon. For example, adjustment and the like are performed so as to prevent the balloon from inflating unevenly in one direction. In addition, air bubbles sometimes enter into the balloon at this time. The air bubbles entered into the balloon are removed by an operation for pushing out the air bubbles to the outside from the ring-shape portions located at the both ends of the balloon.

However, in an ultrasound endoscope used in respiratory fields, etc., for example, an ultrasound transmission/reception portion is particularly small. Therefore, the worker needs to have a high skill to perform the series of operations and such operations have been very difficult.

The present invention has been achieved in view of the above-described points, and an object of the present invention is to provide an ultrasound probe capable of improving workability of mounting a balloon and removing air bubbles and the like after the mounting of the balloon.

WO 2008/115151 A1 discloses an ultrasound probe assembly comprising a sigmoidoscope, an ultrasound probe, an inflatable membrane positioned over the probe and a water stand. The inflatable membrane is secured at the water stand with two rubber rings.

WO 2011/028181 A1 discloses an anorectal probe assembly comprising an anorectal probe with an ultrasound transducer and a balloon mounted to the probe. The probe has an enlargement between the handle and the shaft.

### Disclosure of Invention

### Means for Solving the Problem

In order to achieve the above-described object, an ultrasound probe according to claim 1 is provided.

According to the present invention, it is possible to provide an ultrasound probe capable of improving workability of mounting a balloon and removing air bubbles and the like after the mounting of the balloon.

### Brief Description of the Drawings

FIG 1 is a schematic configuration diagram showing a whole of an ultrasound endoscope system including an ultrasound probe according to a first embodiment of the present invention.
FIG 2 is a schematic perspective view showing, in an enlarged manner, a schematic configuration of a part near a distal end section of the ultrasound probe in the ultrasound endoscope system in FIG. 1.
FIG. 3 is a schematic projection view of FIG. 2.
FIG. 4 is a schematic perspective view showing a state where a balloon is mounted to the distal end section of the ultrasound probe in FIG. 2.
FIG. 5 is a schematic projection cross-sectional view of FIG. 4.
FIG. 6 is a schematic perspective view showing a state where a balloon fixing tool is mounted to the distal end section (in the balloon mounted state) of the ultrasound probe in FIG. 4.
FIG. 7 is a schematic projection cross-sectional view of FIG. 6.
FIG. 8 is an enlarged cross-sectional view of a site shown by the reference numeral [8] in FIG. 7.
FIG. 9 is a schematic perspective view showing a modified example of the balloon fixing tool in the ultrasound probe according to the first embodiment of the present invention.
FIG. 10 is a schematic perspective view showing another modified example of the balloon fixing tool in the ultrasound probe according to the first embodiment of the present invention.
FIG 11 is a schematic view showing a schematic configuration of a part near a distal end section of an ultrasound probe according to a second embodiment of the present invention.
FIG. 12 is a schematic perspective view showing a schematic configuration of a part near a distal end section of an ultrasound probe according to a third embodiment of the present invention.
FIG. 13 is a main-part enlarged perspective view showing the balloon fixing tool in the ultrasound probe in FIG. 12 in an enlarged manner by taking out only the balloon fixing tool.
FIG. 14 is a schematic projection cross-sectional view showing a state where the balloon and the balloon fixing tool are mounted to the distal end section of the ultrasound probe in FIG. 12.
FIG. 15 is an enlarged cross-sectional view of a site shown by the reference numeral [15] in FIG. 14.
FIG. 16 is a schematic view showing a schematic configuration of a part near a distal end section of an ultrasound probe according to a fourth embodiment of the present invention.
FIG. 17 is a schematic perspective view showing a state where the balloon is mounted to the distal end section of the ultrasound probe in FIG. 16.
FIG. 18 is a cross-sectional view showing a schematic configuration of a part near a balloon mounting and fixing portion at the distal end section of the ultrasound probe in FIG. 16.

### Best Mode for Carrying Out the Invention

Hereinafter, the present invention will be described with embodiments shown in the drawings. Note that, in the drawings used for the explanation below, there is a case where scale sizes are different for each of the constituent elements such that the respective constituent elements have sizes recognizable in the drawings. Therefore, in the present invention, the number, the shapes, the ratio of the sizes of the constituent elements, and the relative positional relationship among the respective constituent elements shown in the drawings are not limited to those in the configurations shown in the drawings.

### [First Embodiment]

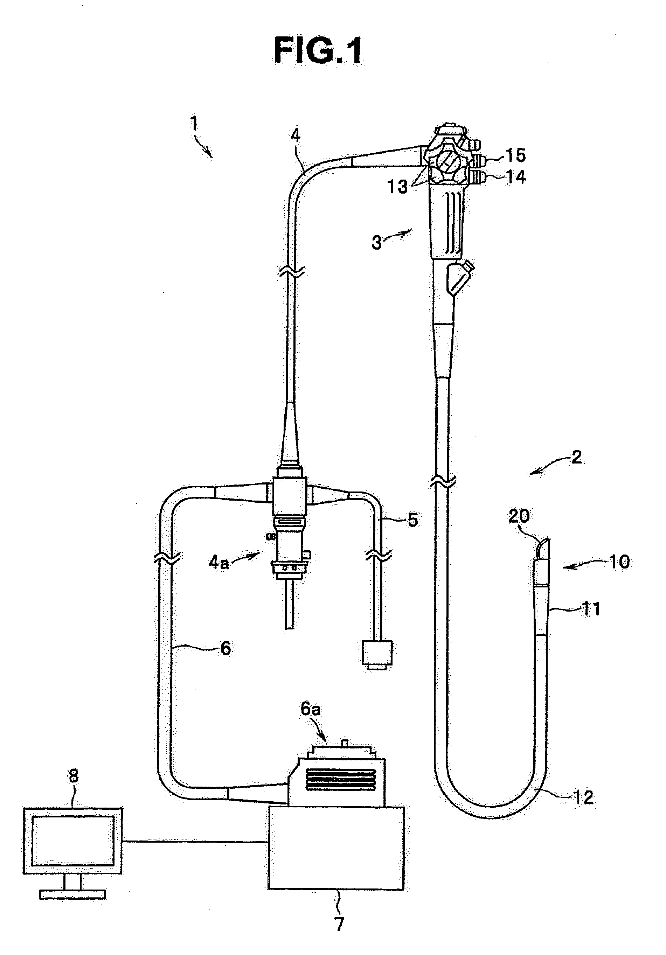

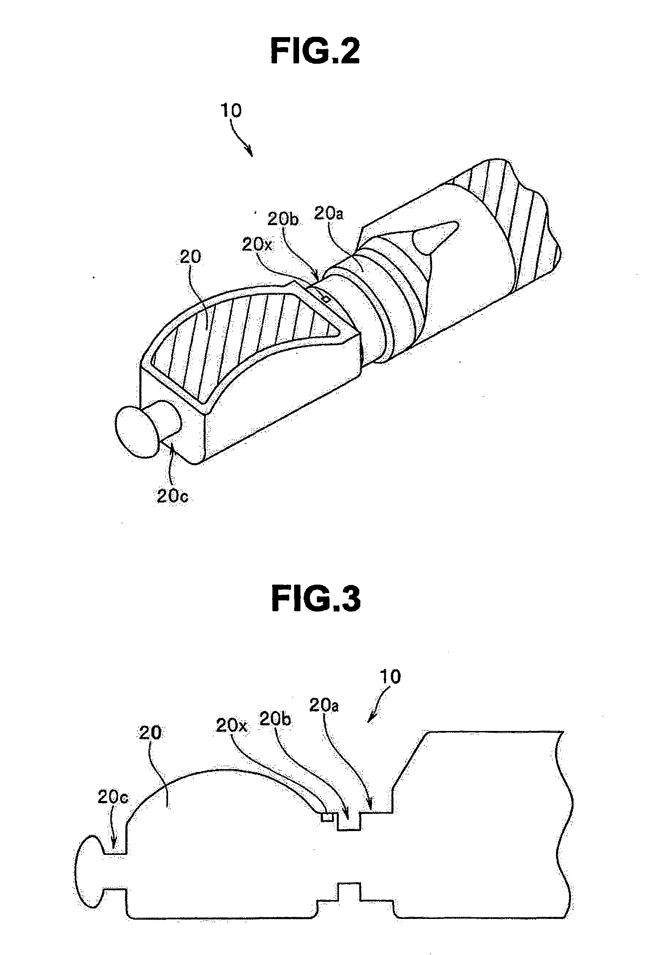

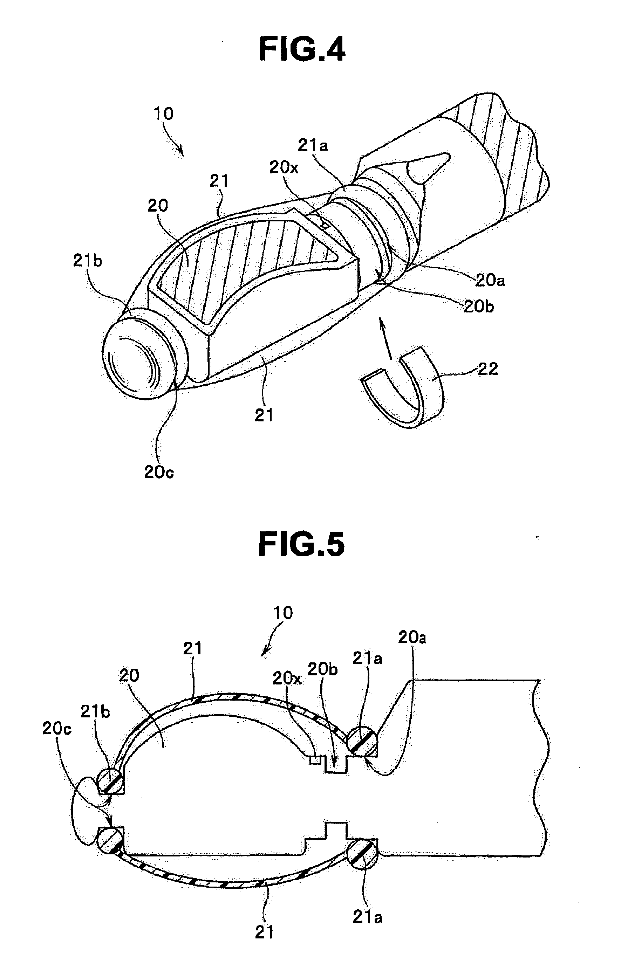

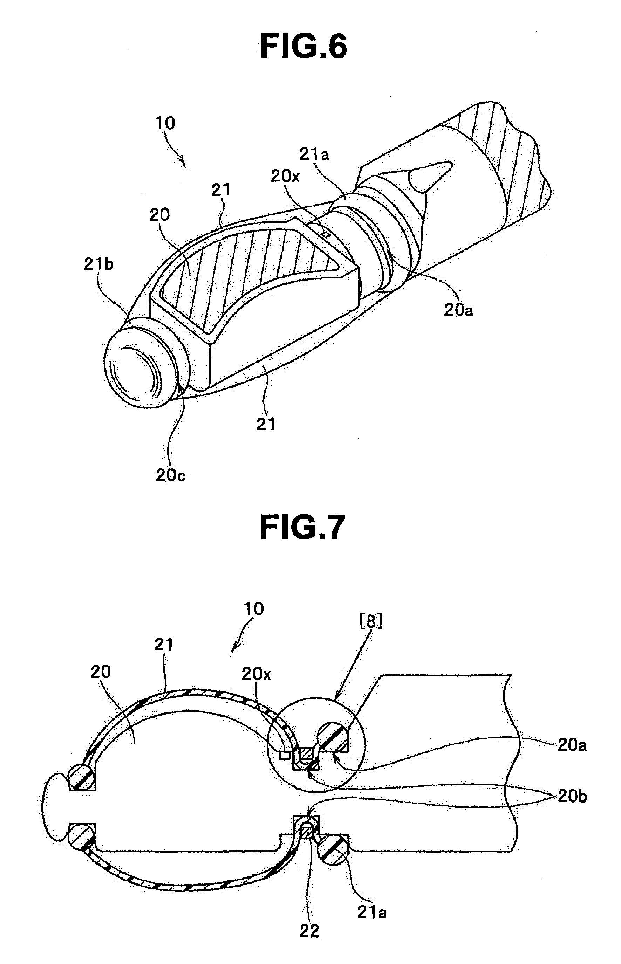

FIGS. 1 to 8 describe the first embodiment of the present invention. Note that, in the schematic projection view in FIG. 3, only the visible outline at the time that the vicinity of a distal end section of an ultrasound probe is projected is shown, and illustration of an internal configuration is omitted in order to avoid complication of the drawing. Further, FIG. 5 shows a cross section of a balloon in the mounted state, in addition to the schematic projection view of FIG. 3. Furthermore, FIG. 7 shows the state in FIG. 5, that is, the state where a balloon fixing tool is mounted to a predetermined site near the distal end section of the ultrasound probe to which the balloon is mounted, and shows the balloon and the balloon fixing tool in the cross sections thereof.

First, the whole configuration of the ultrasound endoscope system including the ultrasound probe according to the first embodiment of the present invention will be described below with reference to FIG 1.

The ultrasound endoscope system including the ultrasound probe according to the present embodiment is configured by mainly including an ultrasound endoscope 1, an ultrasound observation control apparatus 7, a display device, and the like.

The ultrasound endoscope 1 is configured mainly by an insertion section 2 configured to be able to be introduced into a body of a subject, an control section 3 provided in a linked manner on the proximal end side of the insertion section 2, and a universal cord 4 extended from a side portion of the control section 3.

The insertion section 2 is configured by including: a distal end section 10 disposed at the distal end of the insertion section; a bending section 11 which is formed to be bendable and provided in a linked manner on the proximal end side of the distal end section 10; and a flexible tube portion 12 which has flexibility and which is provided in a linked manner on the proximal end side of the bending section 11 and connected to the distal end side of the control section 3. Note that the ultrasound endoscope 1 may be configured as what is called a rigid endoscope in which the insertion section 2 is made of a rigid member.

An ultrasound probe 20, which includes an ultrasound transmission/reception portion configured to transmit and receive ultrasound and a case member configured to hold the ultrasound transmission/reception portion, and the like, is provided at the distal end section 10 of the insertion section 2.

The control section 3 is provided with an angle operation knob 13 for operating bending of the bending section 11. Further, the control section 3 is provided with valves 14, 15 for controlling a feeding operation and sucking operation of fluid from an opening portion provided at the distal end section 10.

An endoscope connector 4a configured to be connected to a light source device, not shown, is provided at the proximal end portion of the universal cord 4. A video cable 5, an ultrasound cable 6, and the like are extended from the endoscope connector 4a. The ultrasound cable 6 is detachably connected to the ultrasound observation control apparatus 7 through an ultrasound connector 6a. The ultrasound observation control apparatus 7 is a control apparatus which controls transmission and reception operations of ultrasound by the ultrasound transmission/reception portion 20, generates an ultrasound image, and outputs the generated ultrasound image to an image display device 8. The ultrasound observation control apparatus 7 is also called as an ultrasound observation apparatus.

As shown in FIGS. 2 and 3, the distal end section 10 is provided with the ultrasound probe 20. The case member that holds the ultrasound probe 20 is configured such that an ultrasound balloon 21 can be mounted and fitted thereto, as shown in FIGS. 3 and 4.

The ultrasound balloon 21 is disposed at the case member of the ultrasound probe 20 so as to cover the ultrasound transmission/reception portion with an elastic member. When the ultrasound balloon 21 is in a mounted state with respect to the ultrasound probe 20, an ultrasound transmission medium is filled inside the ultrasound balloon 21.

To this end, the ultrasound balloon 21 is formed by including, at both ends of the cylindrical-shaped elastic member, thick ring-shape portions, that is, opening portions composed of a proximal end ring-shape portion 21 a and a distal end ring-shape portion 21b, for example.

In accordance with the configuration, as shown in FIGS. 2 to 5, the ultrasound probe 20 is provided with a distal-end-side balloon fixing portion 20c to which the distal end ring-shape portion 21b of the ultrasound balloon 21 is fitted, a proximal-end-side balloon fixing portion 20a to which the proximal end ring-shape portion 21 a is placed and fixed, and a balloon fixing tool mounting groove 20b into which a balloon fixing tool 22 (see FIG. 4) to be described later is fitted. In this case, the distal-end-side balloon fixing portion 20c is a circumferential groove formed on the distal-most side of the ultrasound balloon 21. In addition, the proximal-end-side balloon fixing portion 20a is formed at a site on a more proximal end side than the ultrasound transmission/reception portion. The proximal-end-side balloon fixing portion 20a is formed such that the cross-sectional shape thereof has a stepped shape in which the proximal end side is made higher, as shown in FIG. 3.

The balloon fixing tool mounting groove 20b is a circumferential groove formed at a predetermined site of the case member of the ultrasound probe, that is, a site between the ultrasound transmission/reception portion and the proximal-end-side balloon fixing portion 20a. The balloon fixing tool mounting groove 20b is formed in a groove shape into which the balloon fixing tool 22 for fixing the ultrasound balloon 21 by pressing a part of the ultrasound balloon can be fitted, as described above. Note that the balloon fixing tool mounting groove 20b is formed to have a width narrower than the width of the proximal-end-side balloon fixing portion 20a or the proximal end ring-shape portion 21a. That is, the proximal-end-side balloon fixing portion 20a has a width wide enough to place thereon the proximal end ring-shape portion 21 a. On the other hand, the balloon fixing tool mounting groove 20b is set to have a narrow width to the extent that the proximal end ring-shape portion 21 a does not fall into the groove.

The balloon fixing tool 22 is formed in a circular ring shape with a part thereof being cut out (what is called, C-ring shape), for example, and has elasticity in the circumferential direction. The balloon fixing tool 22 is detachably disposed in the balloon fixing tool mounting groove 20b by holding a part of the ultrasound balloon 21, as described above, that is, holding the outer circumference between the both ring-shape portions 21 a and 21b, which is closer to the proximal end ring-shape portion 21a. Note that the balloon fixing tool 22 is formed such that the cross section thereof has a shape which is at least more than a semicircle. In addition, in accordance with the cross-sectional shape, the site of the case member where the balloon fixing tool 22 is mounted, that is, the site where the balloon fixing tool mounting groove 20b is disposed, is formed such that the cross section thereof is substantially a cylindrical shape. Therefore, when the balloon fixing tool 22 is in a state fitted into the balloon fixing tool mounting groove 20b, the balloon fixing tool 22 is disposed so as to wind around the site where the balloon fixing tool mounting groove 20b is formed, by elastic force of the balloon fixing tool itself, and configured so as not to easily fall off.

Further, since a plurality of types of ultrasound probes having different outer diameters or different lengths exist, it is common that a dedicated balloon is prepared for each of the types. However, conventionally, only a type name has been printed on a packaging bag of a balloon. Accordingly, there has been a possibility to mistake a balloon corresponding to an ultrasound probe for another balloon during operation. Therefore, the balloon fixing tool 22 may be provided with an identification portion that is configured to enable identification of the ultrasound probe 20 to which a balloon is fixed. The identification portion is configured as a color code, etc., which can be identified by a color, for example.

An ultrasound transmitting medium inlet 20x, which opens in the same direction as a transmission/reception surface (a shaded area in the site indicated by the reference numeral 20 in FIG. 2), is formed on the case member of the ultrasound probe 20 at a site which is more proximal end side than the ultrasound transmission/reception portion and which is closer to the distal end than the balloon fixing tool mounting groove 20b, in other words, at a site between the ultrasound transmission/reception portion and the balloon fixing tool mounting groove 20b. The ultrasound transmitting medium inlet 20x is connected with an ultrasound transmitting medium inlet tube (not shown) inserted and disposed inside the insertion section 2, the control section 3, and the universal cord 4.

When using the ultrasound probe 20 thus configured according to the present embodiment, the user mounts the ultrasound balloon 21 to the ultrasound probe in advance. Working at that time will be described below.

First, the ultrasound probe 20 is, from the distal end thereof, passed through the opening of the proximal end ring-shape portion 21 a of the ultrasound balloon 21, and the proximal end ring-shape portion 21 a is placed at the proximal-end-side balloon fixing portion 20a. At the same time, the distal end ring-shape portion 21b is fitted to the distal-end-side balloon fixing portion 20c. As a result, the ultrasound balloon 21 is mounted to the case member of the ultrasound probe 20 so as to cover the ultrasound transmission/reception portion of the ultrasound probe 20. The state at this time is shown in FIG 4 and FIG. 5.

In this state, operation for discharging the ultrasound transmitting medium by the control by the ultrasound observation control apparatus 7, for example. According to this operation, the ultrasound transmitting medium passes through the ultrasound transmitting medium inlet tube to be discharged from the ultrasound transmitting medium inlet 20x and injected into the ultrasound balloon 21. The injection operation is stopped when a predetermined amount of ultrasound transmitting medium is filled in the ultrasound balloon 21. At this stage, it is checked whether or not air bubbles and the like are mixed in the ultrasound transmitting medium filled in the ultrasound balloon 21, whether or not the ultrasound transmitting medium leaks from the ultrasound balloon 21, and whether or not there is a rip on the balloon. When leaking of the ultrasound transmitting medium, a rip on the balloon, or the like is found, the ultrasound balloon 21 is replaced, for example. In addition, when mixture of air bubbles or the like is found, the air bubbles or the like are removed by pinching or turning up the proximal end ring-shape portion 21 a placed at the proximal-end-side balloon fixing portion 20a. Furthermore, it is checked whether or not the ultrasound balloon 21 is inflated unevenly, and if the ultrasound balloon is unevenly inflated, adjustment is performed. When such adjustment operations and the like are finished, the balloon fixing tool 22 is fitted into the balloon fixing tool mounting groove 20b. The balloon fixing tool 22 has elasticity in the circumferential direction. Therefore, if the worker puts the balloon fixing tool 22 into the balloon fixing tool mounting groove 20b while applying a force in a direction in which the cut-out portion is opened, and after that, if the worker releases the force, the balloon fixing tool 22 is disposed in a fitted manner so as to grip the balloon fixing tool groove 20b by elasticity of the balloon fixing tool itself. At this time, the balloon fixing tool 22 is fitted into the balloon fixing tool mounting groove 20b by holding a part of the ultrasound balloon 21 (the outer circumference which is between the ring-shape portions 21a, 21b and which is closer to the proximal end ring-shape portion 21a). According to such a configuration, the ultrasound balloon 21 is surely fixed to the case member of the ultrasound probe 20 so as to cover the ultrasound transmission/reception portion of the ultrasound probe 20. The state at this time is shown in FIGS. 6 and 7.

Note that, when it is thus confirmed that the ultrasound probe 20 has been surely mounted in a proper state, operation for withdrawing the ultrasound transmitting medium is performed by the control by the ultrasound observation control apparatus 7. In this state, the ultrasound probe 20 is in an enabled state.

When the ultrasound probe 20 is actually used, the insertion operation of the ultrasound probe 20 is performed in the state where the ultrasound balloon 21 is deflated. When the ultrasound probe 20 reaches in the vicinity of a desired observation target in a body, the ultrasound balloon 21 is brought into an inflated state by injecting the ultrasound transmitting medium, to perform ultrasound observation.

As described above, according to the first embodiment, in the case where the ultrasound balloon 21 is mounted to the ultrasound probe 20 prior to use of the ultrasound probe 20, when mixture of air bubbles or the like in the ultrasound balloon 21 is found and removal operation of the air bubbles or the like is performed, it is possible to rapidly and effectively perform the operation for removing the air bubbles, or the like, since the proximal end ring-shape portion 21 a is placed on the proximal-end-side balloon fixing portion 20a formed in a stepped shape and the operation for pinching or turning up the proximal end ring-shape portion 21 a can be performed extremely easily compared with a conventional structure (structure in which the proximal end ring-shape portion is fitted into a groove).

Then, after such operations, the balloon fixing tool 22 is firmly fitted into the balloon fixing tool mounting groove 20b, which enables the ultrasound balloon 21 to be surely mounted and fixed. Further, even in this state, the proximal end ring-shape portion 21a is in a state placed on the proximal-end-side balloon fixing portion 20a formed in a stepped shape. Therefore, when maintenance such as exchanging of the ultrasound balloon 21 is performed, for example, it is easy to pinch or turn up the proximal end ring-shape portion 21a, thereby capable of easily detaching even the balloon fixing tool 22 which is securely fitted into the balloon fixing tool mounting groove 20b.

In addition, in the conventional structure, the ultrasound balloon 21 has been fixed by pushing the proximal end ring-shape portion 21 a of the ultrasound balloon 21 into the groove portion. However, such an operation process is not required in the structure according to the present embodiment. Therefore, it is possible to simplify the mounting operation of the ultrasound balloon 21.

The balloon fixing tool 22 is provided with a color code associated with the corresponding ultrasound probe 21 so as to be easily identifiable, which makes it possible to surely and unfailingly mount the ultrasound balloon 21 and the balloon fixing tool 22 which are appropriate for the type of the ultrasound balloon 21 to which the ultrasound balloon and the balloon fixing tool are mounted.

The above-described first embodiment exemplifies, as a configuration of the balloon fixing tool 22, the one having elasticity in the circumferential direction and the shape, what is called C-ring shape, formed by cutting out a part of a circular ring shape. The configuration of the balloon fixing tool is not limited to the above, and other various configurations can be considered.

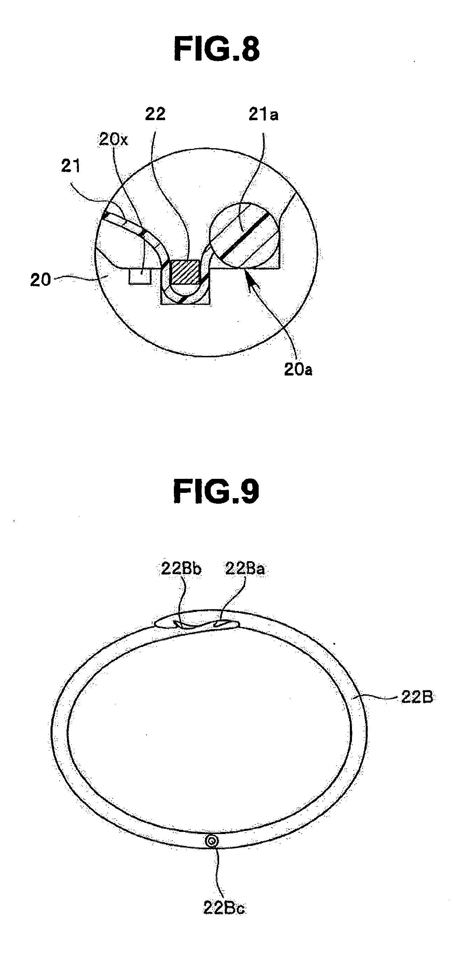

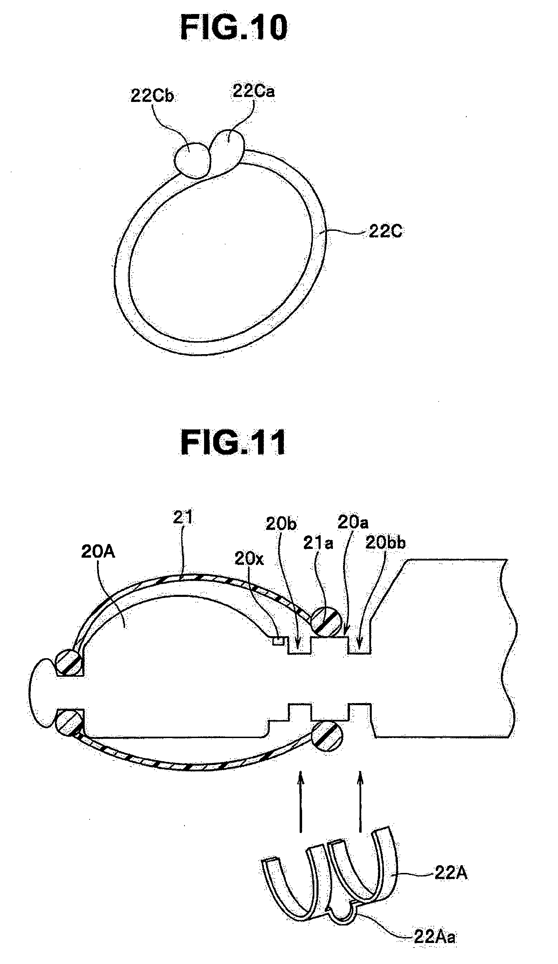

For example, FIGS. 9 and 10 show modified examples of the balloon fixing tool. Each of the balloon fixing tools 22B, 22C shown in FIGS. 9 and 10 is formed by cutting off a part of a member, the whole shape of which is a circular ring shape, and includes an engagement mechanism at the cut-off sites. Note that the cross-sectional shapes of the balloon fixing tools 22B, 22C in this case are substantially circular shapes, for example.

The balloon fixing tool 22B in FIG. 9 is configured such that one ends of two semicircular members formed by dividing a circular ring-shaped member into two are rotatably connected to each other and the other ends, that is, two opposed end portions of the cut-off sites are provided with engaging pawls 22Ba, 22Bb, respectively.

When the engaging pawls 22Ba, 22Bb are engaged with each other, the whole shape becomes a circular ring shape. In addition, when the engaged state between the engaging pawls 22Ba and 22Bb is released, the two semicircular members rotate at a connecting portion 22Bc such that the opposed two end portions of the cut-off sites are separated from each other. Therefore, the cut-off sites can be separated from each other only by rotating the two semicircular members with the connecting portion 22Bc as a rotational center, which enables the fitting operation of the balloon fixing tool into the balloon fixing tool mounting groove 20b to be performed easily.

Further, the balloon fixing tool 22C in FIG. 10 is formed by cutting off a part of a circular ring-shaped member and forming convex portions 22Ca, 22Cb respectively at two opposed end portions at the cut-off sites. The balloon fixing tool 22C is formed so as to have elasticity in the circumferential direction.

The balloon fixing tool, the whole shape of which is circular ring shape, is formed by engaging the convex portions 22Ca and 22Cb with each other. In addition, the balloon fixing tool can be fitted into the balloon fixing tool mounting groove 20b by pressing the cut-off sites apart against the elasticity in the circumferential direction in the state where the engagement between the convex portions 22Ca and 22Cb is released.

As a result, also when these balloon fixing tools 22B and 22C are used, the same effects as those in the above-described first embodiment can be obtained.

Note that, as the balloon fixing tool 22 in the above-described first embodiment, the one having a substantially rectangular cross-section is exemplified as shown in the drawings (see FIGS. 4 and 7). In addition, though not clearly shown, the above-described two modified examples (FIGS. 9, 10) exemplify the balloon fixing tool supposing that the tool has a substantially circular cross section. However, the cross-sectional shape of the balloon fixing tool according to the present invention is not limited to the above-described examples. That is, the balloon fixing tools, cross sections of which are various shapes such as a substantially circular shape, an elliptic shape, or a polygonal shape, can be also used.

### [Second Embodiment]

FIG. 11 is a schematic view showing a schematic configuration of a part near a distal end section of an ultrasound probe according to a second embodiment of the present invention. Note that, also in FIG 11, only the visible outline at the time that the vicinity of the distal end section of the ultrasound probe is projected is shown, illustration of an internal configuration is omitted, and only the mounted balloon is shown in the cross section thereof, in order to avoid complication of the drawing. Further, the balloon fixing tool to be mounted to the ultrasound probe is shown in perspective view.

The present embodiment has substantially the same configurations as those in the first embodiment. However, an ultrasound probe 20A according to the present embodiment is different from the one in the first embodiment in that the two balloon fixing tool mounting grooves 20b, 20bb are provided and a balloon fixing tool 22A having a shape corresponding to the grooves is used.

As described above, the two balloon fixing tool mounting grooves 20b, 20bb are provided on the proximal end side of the ultrasound probe 20A according to the present embodiment, and the proximal-end-side balloon fixing portion 20a is formed between the two balloon fixing tool mounting grooves 20b and 20bb. The groove widths of the two balloon fixing tool mounting grooves 20b, 20bb are set so as to be narrower than the thickness (diameter) of the proximal end ring-shape portion 21 a of the ultrasound balloon 21.

On the other hand, the balloon fixing tool 22A is formed such that members having what is called C-ring shape which is substantially the same shape as that of the balloon fixing tool 22 in the first embodiment are connected to each other at a connecting portion 22Aa. Furthermore, the connecting portion 22Aa includes a convex-shaped portion formed outwardly such that the connection portion 22Aa does not interfere with the proximal end ring-shape portion 21 a of the ultrasound balloon 21 when the balloon fixing tool 22A is attached to a predetermined position of the ultrasound probe 20A, that is, in the state where the two C-ring shaped portions are fitted into the two balloon fixing tool mounting grooves 20b, 20bb. Other configurations and workings are the same as those in the above-described first embodiment.

As described above, according to the second embodiment, the same effects as those in the first embodiment can be obtained. In addition, in the present embodiment, the balloon fixing tool 22A is configured to have the two C-ring shaped portions, thereby enabling attachment and detachment of the balloon fixing tool 22A to be performed more easily.

### [Third Embodiment]

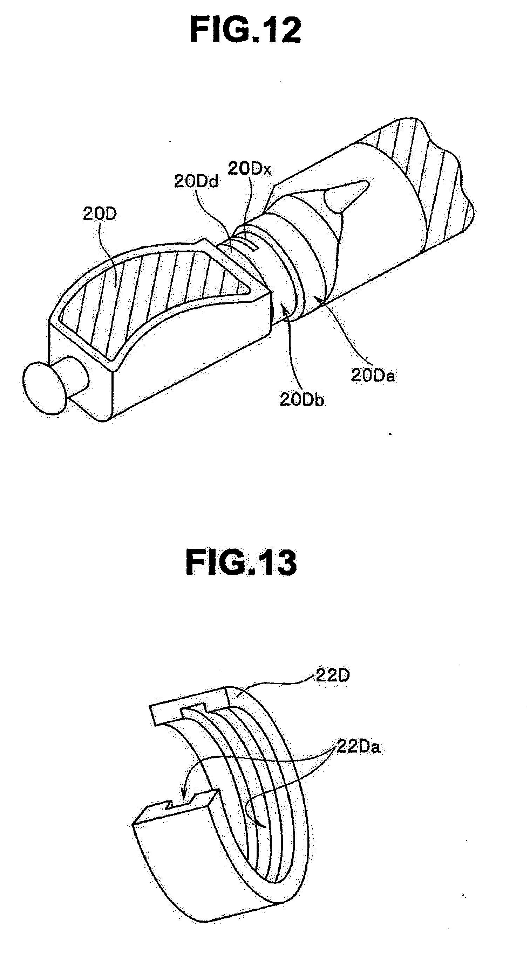

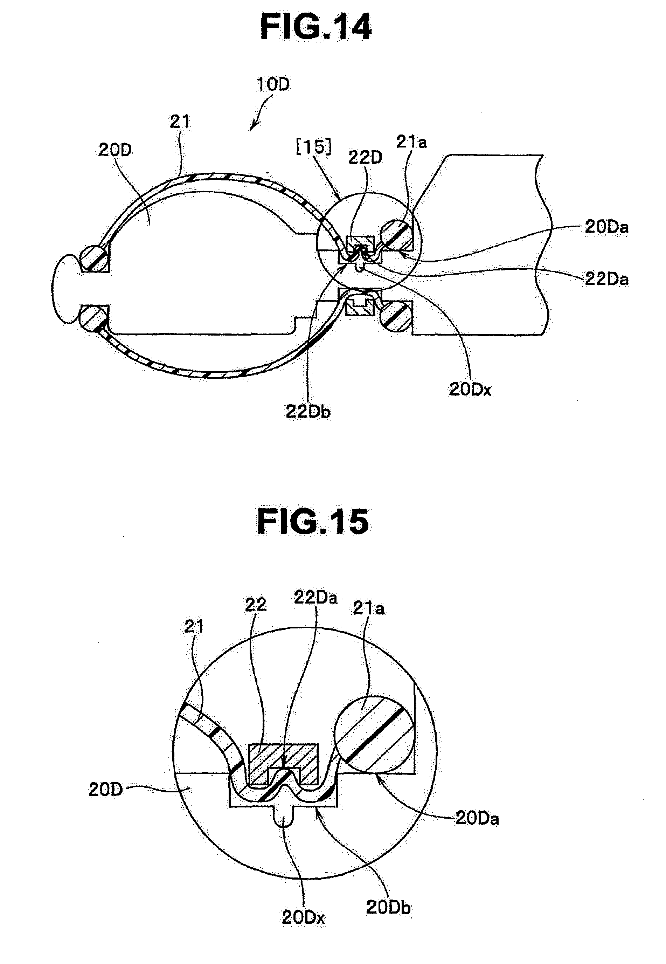

FIGS. 12 to 15 illustrate a third embodiment of the present invention. Note that, in the schematic projection view in FIG. 14, only the visible outline at the time that the vicinity of the distal end section of the ultrasound probe is projected is shown, illustration of an internal configuration is omitted, and only the balloon in the mounted state is shown in the cross section thereof, in order to avoid complication of the drawing.

The present embodiment has substantially the same configurations as those in the first embodiment. However, an ultrasound probe 20D according to the present embodiment is different from the one in the first embodiment in that an ultrasound transmitting medium inlet 20Dx is provided in a balloon fixing tool mounting groove 20Db and a balloon fixing tool 22D having a shape corresponding to the groove is used.

As shown in FIG. 12, the ultrasound transmitting medium inlet 20Dx is provided in the balloon fixing tool mounting groove 20Db of the ultrasound probe 20D according to the present embodiment. In addition, a proximal-end-side balloon fixing portion 20Da is formed at the site closer to the proximal end than the balloon fixing tool mounting groove 20Db. The proximal-end-side balloon fixing portion 20Da is formed such that the cross-sectional shape thereof is a stepped shape in which the proximal end side is made higher, similarly in the first embodiment.

On the other hand, as shown in FIG. 13, the balloon fixing tool 22D is formed such that the whole shape is what is called C-ring shape formed by cutting out a part of a circular ring shape, for example, and has elasticity in the circumferential direction, similarly in the first embodiment. In addition, the balloon fixing tool 22D according to the present embodiment includes, at the substantially center portion on the inner circumferential surface side thereof, a circumferential groove 22Da formed along the circumferential direction. The circumferential groove 22Da is formed at a position opposed to the ultrasound transmitting medium inlet 20Dx of the balloon fixing tool mounting groove 20Db when the balloon fixing tool 22D is fitted into the balloon fixing tool mounting groove 20Db of the ultrasound probe 20D (the state shown in FIG. 14). Furthermore, the circumferential groove 22Da of the balloon fixing tool 22D is formed so as to open toward the distal end side of the ultrasound probe 20D, for example, to allow the ultrasound transmitting medium discharged from the ultrasound transmitting medium inlet 20Dx to be guided toward the ultrasound transmission/reception portion when the injection operation of the ultrasound transmitting medium is performed. Other configurations are substantially the same as those in the first embodiment.

According to such a configuration, in the state where the ultrasound balloon 21 is mounted and fixed by fitting the balloon fixing tool 22D into the balloon fixing tool mounting groove 20Db while sandwiching the ultrasound balloon 21 therebetween, if the injection operation of the ultrasound transmitting medium is performed, the ultrasound transmitting medium causes the sandwiched site of the ultrasound balloon 21 to inflate in the circumferential groove 22Da of the balloon fixing tool 22D, and the ultrasound transmitting medium is smoothly injected into the ultrasound balloon 21. Other workings are substantially the same as that in the first embodiment.

As described above, according to the third embodiment, the same effects as those in the first embodiment can be obtained. In addition, in the present embodiment, the ultrasound transmitting medium inlet 20Dx is provided in the balloon fixing tool mounting groove 20Db and the circumferential groove 22Da is formed on the inner circumferential surface of the balloon fixing tool 22D so as to correspond to the ultrasound transmitting medium inlet. According to such a configuration, when the injection operation of the ultrasound transmitting medium is performed, the ultrasound transmitting medium can be surely injected into the ultrasound balloon 21 and the injection can be effectively performed while preventing the ultrasound balloon 21 from unevenly inflating. Note that, also when discharge operation of the ultrasound transmitting medium is performed, similarly, the balloon can be deflated evenly.

### [Fourth Embodiment]

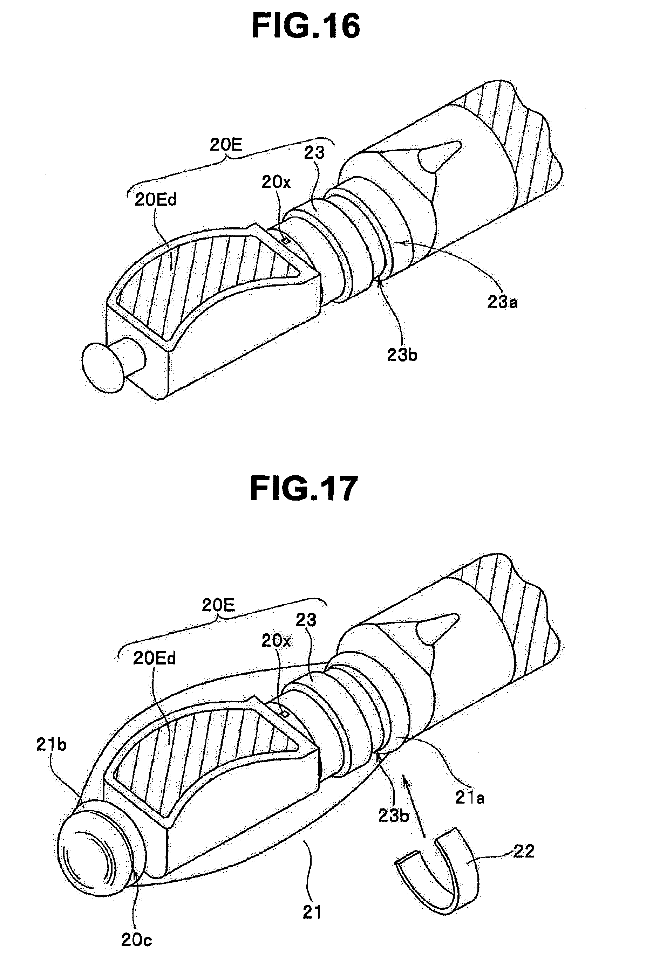

FIGS. 16 to 18 illustrate a fourth embodiment of the present invention. The present embodiment has substantially the same configurations as those in the first embodiment. However, an ultrasound probe 20E according to the present embodiment is different from the one in the first embodiment in that a balloon mounting site adjusting mechanism is provided.

That is, the ultrasound probe 20E according to the present embodiment includes a main body unit 20Ed including an ultrasound transmission/reception portion and a case member, a balloon mounting site adjusting mechanism 23 including a proximal-end-side balloon fixing portion 23 a and a balloon fixing tool mounting groove 23b, and the like.

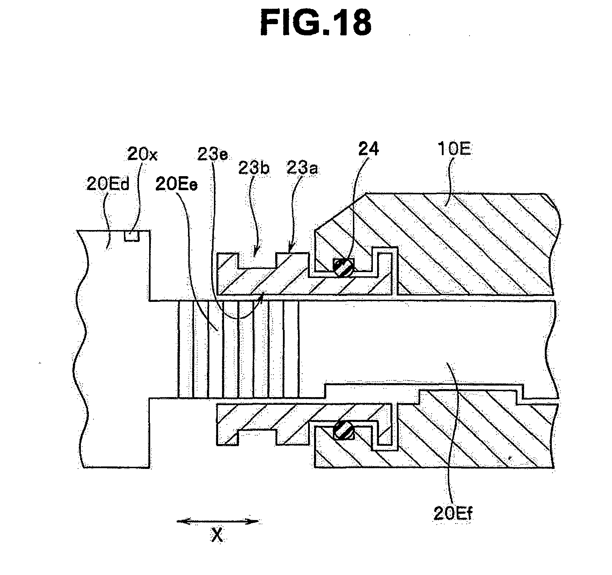

The main body unit 20Ed, though detailed illustration thereof is omitted, includes the ultrasound transmission/reception portion and the case member. As shown in FIG. 18, a pipe 20Ef, which incorporates various signal lines and tubes (not shown), which pass from the distal end opening of the insertion section 2 through inner portions of the insertion section 2, the control section 3, and to the inner portion the universal cord 4, is extended from the proximal end side of the case member. In addition, a screw portion 20Ee is formed on the outer circumference of the pipe 20Ef at a site which is closer to the distal end.

The balloon mounting site adjusting mechanism 23 includes, on the outer circumferential surface thereof, the proximal-end-side balloon fixing portion 23 a and the balloon fixing tool mounting groove 23b, and is a substantially cylindrical member formed as a body separated from the main body unit 20Ed. In addition, as shown in FIG. 18, a female screw portion 23e which screws with a screw portion 20Ee of the pipe 20Ef is formed on the inner circumferential surface of the balloon mounting site adjusting mechanism 23. The balloon mounting site adjusting mechanism 23 is rotatably disposed with respect to the distal-most site of the distal end section 10E. Furthermore, a sealing member 24 is interposed between the balloon mounting site adjusting mechanism 23 and the distal-most site of the distal end section 10E, for example, thereby achieving watertightness. According to such a configuration, when the balloon mounting site adjusting mechanism 23 is rotated around the long axis of the insertion section 2 (distal end section 10E), the main body unit 20Ed and the pipe 20Ef advance or retreat in the direction along the long axis (direction along the arrow X shown in FIG. 18) of the insertion section 2 (distal end section 10E). In this configuration, the arrangement relationship between the proximal-end-side balloon fixing portion 23 a and the balloon fixing tool mounting groove 23b is as follows. That is, the balloon fixing tool mounting groove 23b is disposed at a position closer to the distal end than the proximal-end-side balloon fixing portion 23a.

In addition, the balloon mounting site adjusting mechanism 23 including the balloon fixing tool mounting groove 23b is a pressing portion which is advanceable and retractable on the more distal end side than the proximal-end-side balloon fixing portion 23a, and which presses and fixes the outer circumference of the end portion of the ultrasound balloon 21, which is in the vicinity of the proximal end ring-shape portion 21a and closer to the distal end, using the balloon fixing tool 22. Other configurations are substantially the same as those in the first embodiment.

Also in the ultrasound probe 20E thus configured, the mounting operation of the ultrasound balloon 21 can be performed in substantially the same procedure as that described in the first embodiment.

As described above, according to the fourth embodiment, the same effects as those in the first embodiment can be obtained. Furthermore, the ultrasound probe 20E according to the present embodiment includes the balloon mounting site adjusting mechanism 23. Accordingly, when the mounting operation of the balloon is performed, the balloon mounting site adjusting mechanism 23 having the balloon mounting site on the ultrasound transmission/reception portion side is moved, thereby providing a room in the vicinity of the balloon mounting site in the insertion axis direction. Therefore, it is possible to improve the workability of the operations such as the balloon mounting operation and the air bubbles removing operation.

After the balloon mounting operation, the balloon mounting site adjusting mechanism 23 is moved to the proximal end side, thereby capable of applying tension to the mounted balloon 21. Therefore, it is possible to equalize the tensional force applied to the ultrasound balloon 21, thereby preventing the balloon from unevenly inflating when the ultrasound transmitting medium is injected.

Note that, when the balloon mounting site adjusting mechanism 23 is moved in the state where the balloon is mounted, the balloon mounting site adjusting mechanism 23 only moves inside the ultrasound balloon 21. Therefore, it is possible to prevent outside filth from entering into the ultrasound balloon 21.

The present invention is not limited to the above-described embodiments, and it is needless to say that various modifications and applications can be performed without departing from the scope of the invention. Furthermore, the above-described embodiments include inventions at various stages, and by appropriately combining a plurality of constituent features disclosed in the embodiments, inventions at various stages can also be extracted. For example, even if some constituent features are deleted from all the constituent features shown in the above-described present embodiments, if the problem to be solved by the invention can be solved and the effects of the invention can be obtained, the configuration in which some constituent features are deleted can be extracted as an invention.

### Industrial Applicability

The present invention can be applied not only to endoscope control apparatuses in the medical fields but also in endoscope control apparatuses in the industrial fields.

## Claims

1. An ultrasound probe (20) comprising:
an ultrasound transmission/reception portion which transmits and receives ultrasound;
an ultrasound balloon (21) arranged so as to cover the ultrasound transmission/reception portion, and having at a proximal end thereof an opening and a proximal end ring-shape portion (21a) which borders the opening; and
a proximal-end-side balloon fixing portion (20a, 23a) disposed on a more proximal end side than the ultrasound transmission/reception portion and having a stepped shape in which a proximal end side is high;
**characterized in that** the ultrasound probe (20) further comprises:
a balloon fixing tool mounting groove (20b, 23b) arranged between the ultrasound transmission/reception portion and the proximal-end-side balloon fixing portion (20a, 23a) and having a width in a longitudinal direction of the ultrasound probe (20) narrower than a width of the proximal end ring-shape portion (21a) placed on the proximal-end-side balloon fixing portion (20a, 23a).

2. The ultrasound probe (20) according to claim 1, further comprising:
a balloon fixing tool (22) formed so as to be attachable to and detachable from an outer circumference on a proximal end side of an ultrasound balloon (21), the balloon fixing tool (22) being configured to fix the ultrasound balloon (21) by pressing a part of the ultrasound balloon (21) arranged so as to cover the ultrasound transmission/reception portion.

3. The ultrasound probe (20) according to claim 2, wherein the balloon fixing tool (22) is formed in a shape formed by cutting out a part of a circular ring shape.

4. The ultrasound probe (20) according to claim 2, wherein the balloon fixing tool (22) includes an identification portion configured to be able to identify the ultrasound probe (20) to which the ultrasound balloon (21) is fixed.

## Patentansprüche

1. Ultraschallsonde (20), die umfasst:
einen Ultraschall-Sende/Empfangsabschnitt, der Ultraschall sendet und empfängt;
einen Ultraschallballon (21), der so angeordnet ist, dass er den Ultraschall-Sende/Empfangsabschnitt überdeckt und der an seinem proximalen Ende eine Öffnung und einen ringförmigen proximalen Endabschnitt (21a) aufweist, der an die Öffnung angrenzt; und
einen an einer proximalen Endseite gelegenen Ballonfixierungsabschnitt (20a, 23a), der weiter auf einer proximalen Endseite als der Ultraschall Sende/Empfangsabschnitt angeordnet ist und der eine stufige Form hat, in welcher eine proximale Endseite hoch ist;
**dadurch gekennzeichnet, dass** die Ultraschallsonde (20) weiterhin umfasst:
eine Montagenut (20b, 23b) für ein Ballonfixierungswerkzeug, die zwischen dem Ultraschall-Sende/Empfangsabschnitt und dem an einer proximalen Endseite gelegenen Ballonfixierungsabschnitt (20a, 23a) angeordnet ist und die eine Breite in einer longitudinalen Richtung der Ultraschallsonde (20) hat, die schmäler als eine Breite des ringförmigen proximalen Endabschnitts (21a) ist, der an dem an einer proximalen Endseite gelegenen Ballonfixierungsabschnitt (20a, 23a) angeordnet ist.

2. Ultraschallsonde (20) gemäß Anspruch 1, die weiterhin umfasst:
ein Ballonfixierungswerkzeug (22), das so geformt ist, dass es an einem äußeren Umfang an einer proximalen Endseite des Ultraschallballons (21) befestigbar und davon lösbar ist, wobei das Ballonfixierungswerkzeug (22) dazu eingerichtet ist, den Ultraschallballon (21) durch Drücken eines Teil des Ultraschallballons (21) zu fixieren, der so angeordnet ist, dass er den Ultraschall-Sende/Empfangsabschnitt überdeckt.

3. Ultraschallsonde (20) gemäß Anspruch 2, wobei das Ballonfixierungswerkzeug (22) in einer Form geformt ist, die durch Ausschneiden eines Teils einer Kreisringform geformt wird.

4. Ultraschallsonde (20) gemäß Anspruch 2, wobei das Ballonfixierungswerkzeug (22) einen Identifikationsabschnitt umfasst, der dazu eingerichtet ist, die Ultraschallsonde (20), an der der Ultraschallballon (21) angebracht ist, zu identifizieren.

## Revendications

1. Sonde ultrasonore (20) comprenant :
une partie de transmission/réception d'ultrasons qui transmet et reçoit des ultrasons ;
un ballonnet ultrasonore (21) agencé de façon à couvrir la partie de transmission/réception d'ultrasons, et comportant au niveau d'une extrémité proximale de celui-ci une ouverture et une partie d'extrémité proximale (21a) de forme annulaire qui borde l'ouverture ; et
une partie (20a, 23a) de fixation de ballonnet côté extrémité proximale disposée sur un côté d'extrémité davantage proximale que la partie de transmission/réception d'ultrasons et présentant une forme étagée dans laquelle un côté d'extrémité proximale est élevé ;
**caractérisée en ce que** la sonde ultrasonore (20) comprend en outre :
une rainure (20b, 23b) de montage d'outil de fixation de ballonnet agencée entre la partie de transmission/réception d'ultrasons et la partie (20a, 23a) de fixation de ballonnet côté extrémité proximale et présentant une largeur dans une direction longitudinale de la sonde ultrasonore (20) plus étroite qu'une largeur de la partie d'extrémité proximale (21a) de forme annulaire placée sur la partie (20a, 23a) de fixation de ballonnet côté extrémité proximale.

2. Sonde ultrasonore (20) selon la revendication 1, comprenant en outre :
un outil (22) de fixation de ballonnet formé de façon à être attachable à et détachable d'une circonférence externe sur un côté d'extrémité proximale d'un ballon ultrasonore (21), l'outil (22) de fixation de ballonnet étant configuré pour fixer le ballonnet ultrasonore (21) en pressant une partie du ballonnet ultrasonore (21) agencé de façon à couvrir la partie de transmission/réception d'ultrasons.

3. Sonde ultrasonore (20) selon la revendication 2, dans laquelle l'outil (22) de fixation de ballonnet est formé selon une forme formée en découpant une partie en forme d'anneau circulaire.

4. Sonde ultrasonore (20) selon la revendication 2, dans laquelle l'outil (22) de fixation de ballonnet comprend une partie d'identification configurée pour être capable d'identifier la sonde ultrasonore (20) à laquelle le ballonnet ultrasonore (21) est fixé.
